Europäisches Patentamt

European Patent Office

Office européen des brevets

(19)

(11) Publication number : **0 397 542 B1**

# EUROPEAN PATENT SPECIFICATION

(12)

(45) Date of publication of patent specification :
15.07.92 Bulletin 92/29

(51) Int. Cl.⁵ : **A61K 9/127,** A61K 7/00,
A61K 47/42, A61K 47/36

(21) Application number : 90400967.7

(22) Date of filing : 10.04.90

(54) Stabilization process for hydrated lamellar phases.

(30) Priority : **11.04.89 FR 8904762**
**06.06.89 US 362079**

(43) Date of publication of application :
14.11.90 Bulletin 90/46

(45) Publication of the grant of the patent :
15.07.92 Bulletin 92/29

(84) Designated Contracting States :
AT BE CH DE DK ES GB GR IT LI LU NL SE

(56) References cited :
EP-A- 0 188 821
STN-FILE SEIYER (KARLSRUHE); FILE CHEM-
ICAL ABSTRACTS, vol. 102, abstract no.
209120h, Columbus, Ohio, US; &SEIFEN-
ÖLE-FETTE-WACHSE-JOURNAL FÜR DIE IN-
DUSTRIEPRAXIS, vol. 111, no. 1, 17 January
1985, pages 14-16;
"Alkohol,Glykosaminoglykane Kollagen:
Neue Anwendungen in der Kosmetik"

(56) References cited :
PARFUMS COSMETIQUES AROMES, no. 74,
April/May 1987, page 46, Paris, FR; A. HUC et
al.: "L'utilisation du collagène pour lalibé-
ration prolongée de principes actifs, cas par-
ticulier des liposomes"
CHEMICAL ABSTRACTS, vol. 105, no. 24, 15th
December 1986, page 328, abstract no.
214126f, Columbus, Ohio, US
CHEMICAL ABSTRACTS, vol. 105, no. 24, 15th
December 1986, page 328, abstract no.
214123c, Columbus, Ohio, US
Dorland's Illustrated Medical Dictionary, 26th
Ed., 1981

(73) Proprietor : COLETICA
32, rue Saint Jean de Dieu
F-69007 Lyon (FR)

(72) Inventor : Huc, Alain
26, Chemin des Santons
F-69110 Ste Foy les Lyon (FR)
Inventor : Buffevant, Chantal
Les Carrés - Millery
F-69390 Vernaison (FR)
Inventor : Herbage, Daniel
31, Quai St-Vincent
F-69001 Lyon (FR)

(74) Representative : Portal, Gérard et al
Cabinet Beau de Loménie 55, rue
d'Amsterdam
F-75008 Paris (FR)

## Description

The present invention relates to a stabilization process for hydrated lipid lamellar phases, for example liposomes, to a composition of stabilized hydrated lipid lamellar phases, for example liposomes, and to the use in pharmacy or cosmetology in the form of pharmaceutical or cosmetic compositions.

The main Patent FR-A-2 622 104 has already described a process for stabilizing hydrated lipid lamellar phases, for example liposomes, by introducing the hydrated lipid lamellar phases, or the liposomes, in a homogeneous solution of atelocollagen and glycosaminoglycans.

"Parfums, cosmétiques, arômes", No.74, 1987 describes the stabilization of liposomes by a gel of collagen. According to the invention, a solution of collagen is used.

"Seifen-Öle-Fette-Wachse Journal für die Industrie-praxis", vo.111, no.1, 17 relates to the use in cosmetics of atelocollagen complex and of glycosaminoglycans. No use is described or suggested for stabilizing liposomes.

According to the present invention, it has now been unexpectedly discovered that the stabilization of the hydrated lipid lamellar phases, particularly liposomes, might be increased further when the hydrated lipid lamellar phases, or the liposomes, were manufactured in the presence of such a homogeneous solution of atelocollagen-glycosaminoglycan or atelocollagen-mucopolysaccharide.

According to a first aspect, the present invention thus provides a stabilization process for hydrated lipid lamellar phases, or vesicles of the liposome type, characterized in that the hydrated lipid lamellar phases, or the vesicles of the liposome type, are prepared in the presence of a homogeneous solution of atelocollagen-glycosaminoglycan or atelocollagen-mucopolysaccharide.

The homogeneous solution of atelocollagen and of glycosaminoglycan or mucopolysaccharide is advantageously such as prepared in the main Patent which is incorporated herein by reference.

According to an advantageous variant embodiment, the encapsulated active ingredient is dissolved in the solution of atelocollagen-glycosaminoglycan or atelocollagen-mucopolysaccharide. Any active ingredient may be used. Preferred active ingredients are cosmetic or pharmaceutical active ingredients. For example, heparan sulfate may be used as active ingredient.

According to a second aspect, the present invention also provides a composition containing stabilized hydrated lipid lamellar phases, or vesicles of the liposome type, characterized in that the hydrated lipid lamellar phases, or the vesicles of the liposome type, were prepared in the presence of a homogeneous solution of atelocollagen and glycosaminoglycan or atelocollagen-mucopolysaccharide.

As described in the main Patent, the compositions according to the invention are preferably used in pharmacy or in cosmetology, in the form of pharmaceutical or cosmetic compositions. To that end, the composition may be used as such, or various suitable constituents or excipients, well known to the man skilled in the art, may be added thereto without any particular problem, provided that it is established that such addition does not modify the stability of the hydrated lipid lamellar phases, particularly vesicles of the liposome type.

Thanks to the invention, a very great stability of the vesicles of the liposome type, is unexpectedly obtained. A diminution of the antigenicity of the stabilizing support is also obtained, as well as a marked improvement in the protection of one of the constituents of the stabilizing support, namely the atelocollagen towards collagenases which leads to an "in vivo" prolongation of the retarding effect of the atelocollagen.

The compositions according to the invention present an increased hydrating property, a more marked action of regeneration of the dermis and the epidermis by increase of cellular development.

The other advantage of the invention resides in that the process for manufacturing vesicles of the liposome type is extremely simple, also enabling emulsions to be prepared.

The invention therefore brings determining noteworthy technical improvements over the prior art.

Other aims, properties and advantages of the invention will also be clearly apparent in the light of the following explanatory description made with reference to an example of compositions according to the invention employing the process according to the invention described previously with a test of stability.

EXAMPLE 1

Preparation of a composition of liposomes containing heparan sulfate as active ingredient in the presence of a complex solution of atelocollagen-glycosaminoglycans

10 kg of atelocollagen-glycosaminoglycan complex, containing 20 g of collagen, 5 g of chondroitin 4-sulfate are prepared as described in steps b to d of Example 1 of FR-A-2 622 104. To this solution may be added conserving agents, for example 10 g of Nipagin® and 50 g of Phenonip® manufactured by NIPA Laboratories Limited (UK). In this solution are added 10 g of heparan sulfate with mechanical stirring until a homogeneous solution is obtained. The pH is adjusted to 7.2 with sodium hydroxide.

In this bath maintained with stirring at ambient temperature, 100 g of egg white lecithin are added. After complete dissolution, the mixture is agitated by an ultrasonic stirrer of the Ultra-Turax UTL 60 type, rotating at 8000 rpm for 10 mins.

10 kg of solution containing stabilized liposomic vesicles in the atelocollagen-glycosaminoglycan solution are thus obtained.

The stability of the liposomes is checked by electronic transmission microscope as described in Example 2.

## EXAMPLE 2

Stability of the liposomes obtained in Example 1.

The stability of the liposomes obtained in Example 1 is checked by studying the permeability of the membrane of the liposomic vesicles with respect to 6-carboxyfluorescein (6-CF).

To that end, the 6-carboxyfluorescein which has been added in the atelocollagen-glycosaminoglycan solution, is encapsulated in the liposomic vesicles.

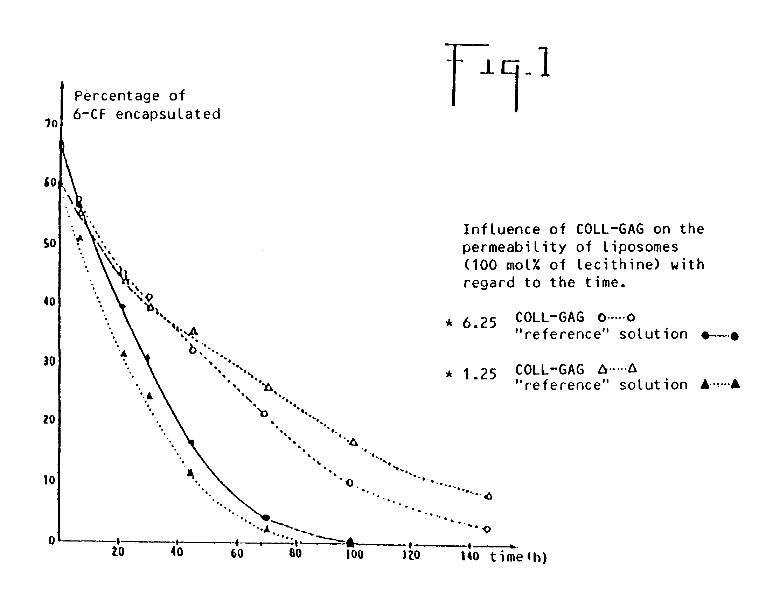

Liposomes encapsulating the 6-carboxyfluorescein were firstly prepared in conventional manner by ultrasonic agitation, the liposomes obtained therefore being non-stabilized, called "control" solution, Fig. 1, in which is introduced a volume of dilution identical to the volume of stabilizing collagen-glycosaminoglycan support, abbreviated to COLL-GAG, having the same pH and the same osmotic pressure, the "control" solution curve being made for two different values of the lipid concentration/collagen concentration ratio, equal respectively to 6.25 ("control" solution curve ● - ●) or to 1.25, curve ▲ - ▲ , Fig. 1, with respect to liposomes prepared by ultrasonic agitation in conventional manner but stabilized thereafter by the introduction of the latter in an atelocollagen-glycosaminoglycan solution (cf. curves COLL-GAG O-O Δ-Δ , Fig. 1).

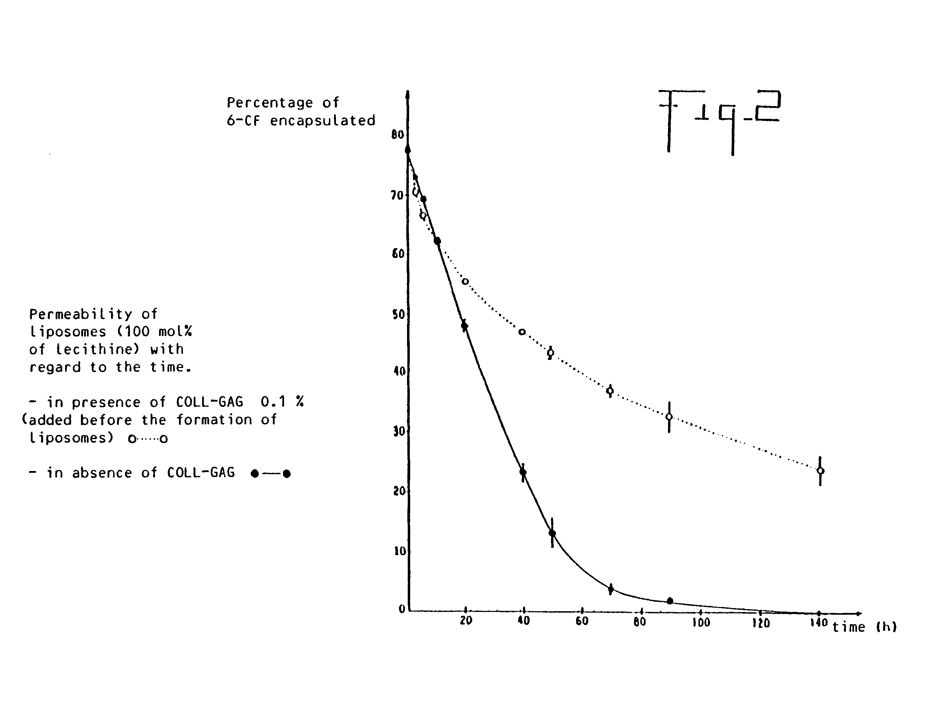

Furthermore, Fig. 2 shows the curves of permeability of the liposomes (100 mol % of lecithin) in the course of time, viz. when the liposomes are prepared in the presence of the 0.1% atelocollagen-glycosaminoglycan solution according to the present invention (curve O-O, Fig. 2) with respect to the "control" solution (in the absence of COLL-GAG) (curve ●-●, Fig. 2).

An examination of Figs. 1 and 2 very clearly shows that the permeability of the liposomic wall is reduced in the case of the liposomes stabilized by the solution of collagen-GAG after formation of the capsules with respect to the non-stabilized liposomes (cf. Fig. 1). This phenomenon is marked still more when the liposomes have been formed in the presence of collagen-GAG (Fig. 2).

## Example 3

### Pharmaceutical or cosmetic composition

A composition prepared as in example 1 can be used as a pharmaceutical or cosmetic composition. Quite naturally, in such a case it is usually necessary to encapsulate an active ingredient in the liposomes, either in the membrane of the liposome or in the interior of the liposomes depending on whether the active substance is hydrophobic or hydrophilic in nature, according to the procedure which is, moreover, cited in example 1 at the end of paragraph a) of FR-A-2622 104 and is well known to the person skilled in the art.

Various excipients or other active components may be added as desired, provided that they do not destroy the stabilizing effect of the support according to the invention, as can be readily understood.

### Example of a composition in an emulsified form

This composition has the following empirical formulation, the percentages being expressed by weight :

| | % by weight |
|---|---|
| Polyoxypropylene 15 (POP) - Stearyl alcohol | 4 |
| Sodium 2-stearoyl lactate | 4 |
| Polyoxy ethylene fatty acid ester | 3 |
| Glycerol stearate | 1 |
| Dioctonate of polypropylene glycol | 2 |
| Glycerol stearate | 2 |
| Methyl parabenzoate | 0.3 |
| Polypropylene glycol | 2 |
| Allantoin | 0.2 |
| Carboner 940[R] | 0.2 |
| Triethanolamine | 0.5 |
| "Complex" of liposomes in the atelocollagen-glycosaminoglycan stabilizing support according to the invention | 30 |
| Purified water | 50.8 |
| | 100 % |

The preparation of this composition in emulsified form is carried out in the following manner.

First, an emulsion is prepared in purified water of all of the components, other than the "complex" of liposomes in the atelocollagenglycosaminoglycan stabilizing support according to the invention, in a standard manner.

Once this emulsion is formed, the "complex" of liposomes in the atelocollagen-glycosaminoglycan stabilizing support according to the invention, such as that prepared according to examples 1 or 2, is added with stirring which is maintained for 1 hour, care being taken to maintain the temperature below 30°C.

In this way, a composition in emulsified form is obtained in which liposomes are stable.

This stability was checked by electron microscopy and constitutes a remarkable result of the invention.

Naturally, the present invention comprises all of the agents constituting technical equivalents of the agents described as well as their various combinations. For example, it is quite clear that the term "glycosaminoglycan" need not be interpreted strictly and that it includes the mucopolysaccharides as equivalents, given that the glycosaminoglycans are polymers constituted of disaccharide units arranged in a linear manner and usually composed of an uronic acid and a hexosamine. Thus, the mucopolysaccharides are included in the definition of the glycosaminoglycans.

Similarly, the atelocollagen must be understood as being collagen from which the telopeptides have been removed and which constitute uncross-linked collagen as understood by the person skilled in the art.

Finally, it is to be observed that the combination according to the invention of glycosamoglycans and atelocollagen makes it possible to prepare a stabilizing support in the form of a solution at a pH close to neutrality without precipitation of the atelocollagen being brought about. Furthermore, the support according to the invention makes it possible to prepare emulsions without difficulty; that constitutes one of the decisive technical advantages of the invention.

In addition, the glycosaminoglycans suppress almost completely the residual antigenicity of the atelocollagen.

It is also to be noted that the complete composition prepared according to the invention may be lyophilized, and this constitutes a crucial industrial advantage.

Finally, the liposome may be manufactured by any method of manufacture of the vesicles of the liposome type compatible with the introduction of the lipids in an atelocollagenglycosaminoglycan solution.

EP 0 397 542 B1

## Claims

### Claims for the following Contracting States : DE, GB, IT, NL, SE, LI/CH, BE, AT, LU, GR, DK

1. A stabilization process for hydrated lipid lamellar phases, or vesicles of liposome type, wherein the hydrated lipid lamellar phases, or the vesicles of liposome type, are prepared in the presence of a homogeneous atelocollagen-glycosaminoglycan or atelocollagen-mucopolysaccharide solution.

2. The process of Claim 1, wherein the hydrated lipid lamellar phases, or the vesicles of liposome type, encapsulate an active ingredient, in particular a cosmetic or pharmaceutical active ingredient.

3. The process of Claim 1 or 2, wherein the active ingredient is dissolved in the solution of atelocollagen-glycosaminoglycan or atelocollagen-mucopolysaccharide complex.

4. The process of Claim 2 or 3, wherein the active ingredient is heparan sulfate.

5. The process of anyone of Claims 1 to 4, wherein the pH of the solution is close to neutrality.

6. A stabilized composition containing hydrated lipid lamellar phases, or vesicles of liposome type, wherein the hydrated lipid lamellar phases, or vesicles of liposome type, have been prepared in the presence of a solution of atelocollagen and of glycosaminoglycan or atellocollagen mucopolysaccharide, in accordance with the process described in one of Claims 1 to 5.

7. Use of the composition as defined in Claim 6 or as obtained by the process according to one of Claims 1 to 5, in pharmacy or in cosmetology, in the form of pharmaceutical or cosmetic compositions.

8. Use as defined in claim 7, wherein the composition is under an emulsified form.

9. Use of the composition as defined in claim 7, wherein said composition is lyophilised.

### Claims for the following Contracting State : ES

1. A stabilization process for hydrated lipid lamellar phases,  or vesicles of liposome type, wherein the hydrated lipid lamellar  phases, or the vesicles of liposome type, are prepared in the  presence of a homogeneous atelocollagen-glycosaminoglycan or atelocollagen-mucopolysaccharide solution.

2. The process of Claim 1, wherein the hydrated lipid  lamellar phases, or the vesicles of liposome type, encapsulate an  active ingredient, in particular a cosmetic or pharmaceutical  active ingredient.

3. The process of Claim 1 or 2, wherein the active ingredient  is dissolved in the solution of atelocollagen-glycosaminoglycan or atelocollagen-mucopolysaccharide complex.

4. The process of Claim 2 or 3, wherein the active ingredient  is heparan sulfate.

5. The process of anyone of Claims 1 to 4, wherein the pH of  the solution is close to neutrality.

6. A process of preparation of a pharmaceutical or a cosmetical  composition comprising hydrated lipid lamellar phases, or vesicles  of liposome type prepared in the presence of a solution of  atelocollagen and of glycosaminoglycan or mucopolysaccharide.

7. The process of claim 6, wherein the composition is  prepared under an emulsified form.

8. The process of claim 6, wherein the composition is  prepared under a lyophilised form.

## Patentansprüche

### Patentansprüche für folgende Vertragsstaaten : DE, GB, IT, NL, SE, LI/CH, BE, AT, LU, GR, DK

1. Stabilisierungsverfahren für hydratisierte lamellare Lipidphasen oder Bläschen des Liposomentyps, bei welchem die hydratisierten lamellaren Lipidphasen bzw. die Bläschen des Liposomentyps in Gegenwart einer homogenen Atelocollagen-Glycosaminoglycan- oder -Mucopolysaccharid-Lösung hergestellt werden.

2. Verfahren nach Anspruch 1, bei welchem die hydratisierten lamellaren Lipidphasen bzw. die Bläschen des Liposomentyps ein aktives Ingrediens, insbesondere ein kosmetisches oder ein pharmazeutisches aktives Ingrediens einkapseln.

3. Verfahren nach Anspruch 1 oder 2, bei welchem das aktive Ingrediens in der Lösung des Atelocollagen-Glycosaminoglycan oder -Mucopolysaccharid-Komplexes gelöst wird.

4. Verfahren nach Anspruch 2 oder 3, bei welchem das aktive Ingrediens Heparansulfat ist.

5. Verfahren nach einem der Ansprüche 1 bis 4, bei welchem der pH der Lösung nahezu neutral ist.

6. Stabilisierte Zusammensetzung, enthaltend hydratisierte lamellare Lipidphasen oder Bläschen des Liposomentyps, worin die hydratisierten lamellaren Lipidphasen bzw. die Bläschen des Liposomentyps in Gegenwart einer Lösung von Atelocollagen und von Glycosaminoglycan oder Mucopolysaccharid, insbesondere gemäß dem in einem der Ansprüche 1 bis 5 beschriebenen Verfahren hergestellt sind.

5

7. Verwendung der in Anspruch 6 definierten bzw. durch das Verfahren nach einem der Ansprüche 1 bis 5 erhaltenen Zusammensetzung in der Pharmazie oder in der Kosmetologie in Form von pharmazeutischen oder kosmetischen Zusammensetzungen.

8. Verwendung nach Anspruch 7, wobei die Zusammensetzung in Emulsionsform vorliegt.

9. Verwendung der Zusammensetzung nach Anspruch 7, wobei die Zusammensetzung lyophilisiert ist.

**Patentansprüche für folgende Vertragsstaat : ES**

1. Stabilisierungsverfahren für hydratisierte lamellare Lipidphasen oder Bläschen des Liposomentyps, bei welchem die hydratisierten lamellaren Lipidphasen bzw. die Bläschen des Liposomentyps in Gegenwart einer homogenen Atelocollagen-Glycosaminoglycan- oder -Mucopolysaccharid-Lösung hergestellt werden.

2. Verfahren nach Anspruch 1, bei welchem die hydratisierten lamellaren Lipidphasen bzw. die Bläschen des Liposomentyps ein aktives Ingrediens, insbesondere ein kosmetisches oder ein pharmazeutisches aktives Ingrediens einkapseln.

3. Verfahren nach Anspruch 1 oder 2, bei welchem das aktive Ingrediens in der Lösung des Atelocollagen-Glycosaminoglycan oder -Mucopolysaccharid-Komplexes gelöst wird.

4. Verfahren nach Anspruch 2 oder 3, bei welchem das aktive Ingrediens Heparansulfat ist.

5. Verfahren nach einem der Ansprüche 1 bis 4, bei welchem der pH der Lösung nahe der Neutralität liegt.

6. Verfahren zur Herstellung einer pharmazeutischen oder einer kosmetischen Zusammensetzung, umfassend hydratisierte lamellare Lipidphasen oder Bläschen des Liposomentyps, hergestellt in Gegenwart einer Lösung von Atelocollagen und von Glycosaminoglycan oder Mucopolysaccharid.

7. Verfahren nach Anspruch 6, wobei die Zusammensetzung in Emulsionsform hergestellt wird.

8. Verfahren nach Anspruch 6, wobei die Zusammensetzung in lyophilisierter Form hergestellt wird.

**Revendications**

**Revendications pour les Etats contractants suivants : DE, GB, IT, NL, SE, LI/CH, BE, AT, LU, GR, DK.**

1. Procédé de stabilisation de phases lamellaires lipidiques hydratées, ou de vésicules type liposomes, dans lequel on prépare les phases lamellaires lipidiques hydratées ou les vésicules type liposomes en présence d'une solution homogène d'atélocollagène-glycosaminoglycannes ou mucopolysaccharides.

2. Procédé selon la revendication 1, dans lequel les phases lamellaires lipidiques hydratées ou les vésicules type liposomes encapsulent un principe actif, en particulier un principe actif cosmétique ou pharmaceutique.

3. Procédé selon la revendication 1 ou 2, dans lequel on dissout le principe actif dans la solution de complexe atélocollagène-glycosaminoglycannes ou mucopolysaccharides.

4. Procédé selon la revendication 2 ou 3, dans lequel le principe actif est de l'héparane sulfate.

5. Procédé selon l'une quelconque des revendications 1 à 4, dans lequel le pH de la solution est proche de la neutralité.

6. Composition stabilisée contenant des phases lamellaires lipidiques hydratées ou des vésicules type liposomes, dans laquelle les phases lamellaires lipidiques hydratées ou les vésicules type liposomes ont été préparées en présence d'une solution d'atélocollagène et de glycosaminoglycannes ou de mucopolysaccharides, en particulier selon le procédé décrit dans l'une des revendications 1 à 5.

7. Utilisation de la composition telle que définie à la revendication 6 ou telle qu'obtenue par le procédé selon l'une des revendications 1 à 5, en pharmacie ou en cosmétologie, sous forme de compositions pharmaceutiques ou cosmétiques.

8. Utilisation telle que définie à la revendication 7, dans laquelle la composition se présente sous une forme émulsifiée.

9. Utilisation de la composition telle que définie à la revendication 7, dans laquelle ladite composition est lyophilisée.

**Revendications pour l'Etat contractant suivant : ES.**

1. Procédé de stabilisation de phases lamellaires lipidiques hydratées, ou de vésicules type liposomes, dans lequel on prépare les phases lamellaires lipidiques hydratées ou les vésicules type liposomes en présence d'une solution homogène d'atélocollagène-glycosaminoglycannes ou mucopolysaccharides.

2. Procédé selon la revendication 1, dans lequel les phases lamellaires lipidiques hydratées ou les vésicules type liposomes encapsulent un principe actif, en particulier un principe actif cosmétique ou pharmaceutique.

3. Procédé selon la revendication 1 ou 2, dans lequel on dissout le principe actif dans la solution de complexe atélocollagène-glycosaminoglycannes ou mucopolysaccharides.

4. Procédé selon la revendication 2 ou 3, dans lequel le principe actif est de l'héparane sulfate.

5. Procédé selon l'une quelconque des revendications 1 à 4, dans lequel le pH de la solution est proche de la neutralité.

6. Procédé de préparation d'une composition pharmaceutique ou cosmétique comprenant des phases lamellaires lipidiques hydratées ou des vésicules type liposomes, préparées en présence d'une solution d'atélocollagène et de glycosaminoglycannes ou de mucopolysaccharides.

7. Procédé selon la revendication 6, dans lequel la composition est préparée sous une forme émulsifiée.

8. Procédé selon la revendication 6, dans lequel la composition est préparée sous une forme lyophilisée.

Fig.1

Percentage of
6-CF encapsulated

Influence of COLL-GAG on the
permeability of liposomes
(100 mol% of lecithine) with
regard to the time.

* 6.25   COLL-GAG o·····o
         "reference" solution ●——●

* 1.25   COLL-GAG △·····△
         "reference" solution ▲·····▲

EP 0 397 542 B1

Fig-2

Percentage of
6-CF encapsulated

Permeability of
liposomes (100 mol%
of lecithine) with
regard to the time.

- in presence of COLL-GAG   0.1 %
(added before the formation of
liposomes)   o⋯⋯o

- in absence of COLL-GAG   ●—●

time (h)

EP 0 397 542 B1